# EUROPÄISCHE PATENTANMELDUNG

(11) **EP 2 409 646 A2**
(43) Veröffentlichungstag der Anmeldung: **25.01.2012**
(21) Anmeldenummer: 11172373.0
(22) Anmeldetag: 01.07.2011
(51) Int. Cl.: A61B 6/02, A61B 6/00, A61B 5/107

(54) **Vorrichtung und Verfahren für ein Mammographiegerät**

(30) Priorität: 21.07.2010 DE 102010031740
(71) Anmelder: Siemens Aktiengesellschaft, 80333 München (DE)
(72) Erfinder: Fischer, Daniel, 91052 Erlangen (DE)

(57) **Zusammenfassung**

Mit dieser Vorrichtung und dem dazugehörigen Verfahren werden die maximal möglichen Eckpunkte für eine Trajektorie ermittelt, so dass bei Röntgenaufnahmen das zu durchleuchtende Objekt jeweils vollständig auf dem Detektor abbildbar ist.

## Beschreibung

Eine Erweiterung der klassischen 2-dimensionalen Mammographie stellt die Tomosynthese dar. Hierzu wird z.B. eine Anzahl von 25 Röntgenbildern erstellt. Zu diesen Röntgenaufnahmen wird die Röntgenröhre beispielsweise auf einer Kreisbahn in einem Kreissegment zwischen +25 bis -25 Grad, ausgehend von einer auf der Detektoroberfläche errichteten Senkrechten, verfahren. In regelmäßigen Abständen wird dann eine Röntgenstrahlung in der Röntgenquelle ausgelöst und das jeweilige Röntgenbild vom Detektor ausgelesen und zwischengespeichert. Anschließend wird aus der Vielzahl der in digitalen Bilddaten vorliegenden Projektionen ein Volumensatz mit einem Tomosynthese-Rekonstruktionsprozess erstellt. Je nach Algorithmus erfolgt eine Verrechnung der aus dem Detektor ausgelesenen digitalen Röntgenbilddaten in Time oder nach Abschluss der Röntgenaufnahmen. In dem Volumensatz, können Gewebeveränderungen lokalisiert werden. Gewebeveränderungen unterschiedlicher Größe in einer Brust werden bei verschiedenen Projektionswinkeln unterschiedlich auf den Detektor projiziert. Während der anschließenden Rekonstruktion, z.B. durch die Methode der gefilterten Rückprojektion, werden Gewebestrukturen in der Mama durch geeignetes Filtern, Verschieben und Summieren verstärkt. Die Rekonstruktion führt zu einer Reihe von Schichtbildern in unterschiedlichen Tiefenlagen parallel zur Detektoroberfläche. Eine Betrachtung des Rekonstruktionsergebnisses erfolgt in der Regel in parallel zum Detektor liegenden Z-Schichten des Volumensatzes. Das rekonstruierbare Tomosynthese-Volumen wird durch die Detektorgröße und Auslenkung des Röntgenkopfes beeinflusst. So kann bei einer großen Auslenkung des Röntgenkopfes eine bessere Tiefenauflösung auf Kosten einer Verkleinerung des rekonstruierbaren Volumens erzielt werden. Bei einer kleineren Auslenkung des Röntgenkopfes vergrößert sich das rekonstruierbare Volumen aber die Tiefenauflösung verringert sich dementsprechend.

Bei der Erstellung eines Volumensatzes kann es vorkommen, dass das rekonstruierte Volumen kleiner ist als der eigentlich komprimierte Brustbereich. Dies bringt den Nachteil mit sich, dass keine Diagnose bezüglich des Brustgewebes im Randbereich des Bildes gestellt werden kann.

Der Erfindung liegt die Aufgabe zugrunde, eine Anordnung und ein dazugehöriges Verfahren für eine weitere Ausgestaltung eines Mammographiegerätes anzugeben die den oben genannten Nachteil überwindet.

Die Aufgabe wird durch die in Patentanspruch 1 oder 8 genannten Merkmale gelöst.

Das Mammographiegerät ist dazu derart ausgebildet, dass Eckpunkte der Trajektorie derart festgelegt werden, dass die Röntgenaufnahmen eines Objektes jeweils vollständig vom Detektor erfasst wird, so dass ein aus den Röntgenaufnahmen errechneter Volumensatz das Objekt vollständig abbildet. Um die Eckpunkte der Trajektorie zu ermitteln wird u.a. ein 3D-Modell von dem Objekt erstellt und eine Gerade zwischen Detektor und Röntgenkopf gelegt wobei die Gerade ausgehend vom aktiven Rand des Detektors die Oberfläche des Objektes tangiert. Die Gerade ist ein Platzhalter für einen auf dem Röntgenkegel liegenden Röntgenstrahl. Betrachtet wird dabei der Röntgenstrahl, der bei einer senkrechten Projektion eines verlängerten Trajektorieverlaufes auf dem Röntgenkegel überlagert wird. Zur Ermittlung des 3D-Modells wird beispielsweise ein PMD-Sensor verwendet. Alternativ lässt sich auch ein 3D-Modell aus der Auflagefläche des Objektes auf der Detektoroberfläche während einer Voraufnahme ableiten.

Die Erfindung bringt den Vorteil mit sich, dass bei einer maximalen Auslenkung des Röntgenkopfes abhängig von der Form eines Objektes, dieses bei der Erstellung eines Volumensatzes oder einer sterotaktischen Aufnahme vollständig wiedergegeben werden kann.

Die Erfindung bringt den Vorteil mit sich, dass je Aufnahmezyklus eine maximale Tiefenauflösung erreicht wird.

Die Erfindung bringt den Vorteil mit sich, dass eine manuelle Ermittlung der Eckpunkte der Trajektorie entfällt.

Die Erfindung bringt den Vorteil mit sich, dass die Objektfläche und die Objekthöhe erfasst, die Eckpunkte ermittelt und dadurch die Durchführung des Tomosyntheseverfahrens qualitäts- und zeitoptimiert erfolgt.

Die Erfindung bringt den Vorteil mit sich, dass in Abhängigkeit von einem ermittelten Winkelbereich für eine Trajektorie die Anzahl der Projektionen sowie eine Dosis der Projektion vorgebbar sind.

Die Erfindung wird im Folgenden anhand eines dargestellten Ausführungsbeispieles näher erläutert.

Dabei zeigen:
- Figur 1: eine schematische Darstellung zur Ermittlung von Stellgrößen,
- Figur 2: eine schematische Darstellung zur Ermittlung eines Abschnittes einer Trajektorie und
- Figur 3: ein Blockdiagramm.

Mit dieser Vorrichtung und dem dazugehörigen Verfahren werden die maximal möglichen Eckpunkte für eine Trajektorie ermittelt, so dass bei Röntgenaufnahmen das zu durchleuchtende Objekt jeweils vollständig auf dem Detektor abbildbar ist.

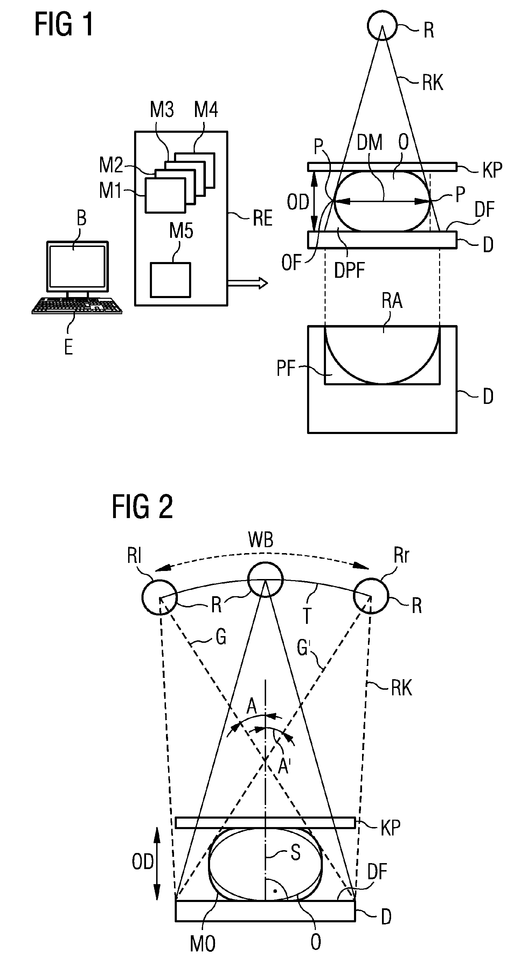

In Figur 1 ist schematisch eine Vorderansicht sowie Teile einer Draufsicht auf eine Röntgenvorrichtung, insbesondere einer Mammographieanlage, wiedergegeben. Die Vorderansicht zeigt eine Röntgenquelle R, eine als Detektor ausgebildete Aufnahmeeinheit sowie eine Kompressionseinheit mit Kompressionsplatte KP und einer dieser gegenüberliegenden Detektoroberfläche DF. Der aus einer Röntgenquelle R ausgehende Röntgenstrahl verläuft z.B. kegelförmig RK. Auf der Oberfläche des Detektors DF wird zu den Röntgenaufnahmen das zur Untersuchung anstehende Objekt O platziert und fixiert. Zur Fixierung eines Objektes O, beispielsweise der Mamma, wird die Kompressionsplatte KP in Richtung Detektoroberfläche DF, überwacht durch Sensoren, manuell oder angetrieben durch einen Motor verschoben. Mit der Röntgenvorrichtung ist auch eine Recheneinheit RE mit einer Eingabe- E und Bildschirmeinheit B verbunden. In der Recheneinheit RE sind die einzelnen Module M1 bis M5 zur Ermittlung des maximal möglichen Neigungswinkels des Röntgenkopfes R bei gleichzeitiger ganzheitlicher Erfassung eines Objektes O angeordnet.

In einem ersten Modul M1 wird der Abstand zwischen Kompressionsplatte KP und Detektoroberfläche DF ermittelt. Liegen zur Tomosynthese bereits eine Voraufnahme vor, so können beispielsweise nach Abschluss der Komprimierung des Objektes O, evtl. unter Einhaltung von bereits erfassten Patientenwerten, der Abstand der Kompressionsplatte KP von der Detektoroberfläche DF mittels Sensoren erfasst werden. Der Durchmesser DM des Objektes O kann über den Abstand zwischen den Punkten P, beispielsweise mit einer optischen oder infrarot Messwerterfassung und Auswertung in Verbindung mit einem Bilderkennungsalgorithmus bzw. Segmentieralgorithmus ermittelt werden.

In einem zweiten Modul M2 wird die Auflagefläche des Objektes O ermittelt. Die Auflagefläche des komprimierten Objektes O wird über Sensoren in der Detektoroberfläche oder an der Unterseite der Kompressionsplatte KP erfasst. Ebenso kann eine Mittelprojektion zur Flächenermittlung herangezogen werden. Die mittlere Querschnittsfläche DM des Objektes kann aufgrund der bekannten Geometrie des Mammographiegerätes und den Gesetzmäßigkeit aus den Strahlensätzen errechnet werden.

In einem dritten Modul M3 ist ein Mittel zur Gewinnung der Form der Oberfläche eines Objektes vorgesehen. Das Mittel ist z.B. ein PMD-Sensor. Das Objekt O wird auf der Auflagefläche platziert und zwischen Auflagefläche und Kompressionsplatte fixiert. Der Sensor tastet das Objekt ab, die Messdaten werden in einer Recheneinheit RE zu einem 3D-Modell umgesetzt.

In einem vierten Modul M4 werden die Daten aus den vorhergehenden Modulen M1, M2 und M3 abgerufen und zu einem kompletten 3D-Model des Objektes O mit Angabe der Position und Orientierung auf der Detektoroberfläche zusammengefügt.

In einem fünften Modul M5, einem Eckpunktermittlungsmodul, werden Eckpunkte Rl, Rr einer Trajektorie T ermittelt. Dazu werden die konstruktiven Eckdaten des Mammographiegerätes, die Daten einer möglichen Trajektorie und das im vierten Modul M4 erstellte 3D- Model mit Position und Orientierung derart verrechnet, dass ausgehend von einer Auslenkung des Röntgenkopfes R eine als Tangente am 3D-Modell anliegende Gerade/Tangente G, G' zwischen Röntgenkopf R oder einem Kollimatorausgang und einem Randbereich des Detektors gezogen wird. Die Gerade/Tangente G, G' bildet in diesem Fall einen äußersten Röntgenstrahl eines von einem Röntgenkopf bzw. von einem steuerbaren Kollimator ausgehenden Röntgenstrahlbündels.

In Figur 2 ist eine schematische Skizze zur Ermittlung eines Auslenkwinkels des Röntgenkopfes R in Abhängigkeit von der Form MO des Objektes O wiedergegeben. Mit dieser schematischen Abbildung soll verdeutlicht werden wie die Form MO des Objektes O einen Einfluss auf die Größe des Winkelbereiches WB der Trajektorie T nimmt. Die Form MO wird durch die Brustdicke OD und Brustfläche OF sowie dem Kompressionsdruck geprägt. Wie in Figur 1 bereits gezeigt und beschrieben, ist auf der Detektoroberfläche DF das Objekt O angeordnet. Das Objekt O wird während der Röntgenaufnahmen mittels der Kompressionsplatte KP auf der Detektoroberfläche DF fixiert. Um ein Volumenbild von dem Objekt O zu erstellen, bedarf es einer Vielzahl von Röntgenaufnahmen. Die digitalen Röntgendaten werden aus der Detektoreinheit D ausgelesen und mittels rekursiver Rechenalgorithmen zu Schichtbildern, beispielsweise in der mit der Mammographieeinheit verbundenen Recheneinheit RE verrechnet. Während eines Zykluses von mehreren Röntgenaufnahmen wird die Röntgenquelle R entlang eines vorgebbaren Kreissegmentes WB auf einer Trajektorie T bewegt. Ein erster und zweiter Eckpunkt Rl, Rr des Kreissegmentes WB der Trajektorie T ist in Figur 2 gezeigt. Betrachtet man den rechten oder zweiten Endpunkt Rr der Trajektorie T, so ergibt sich dieser aus den Randbedingungen derart, dass die äußersten Röntgenstrahlen des Strahlenkegels RK Einerseits vom Randbereich des Detektors D noch empfangen werden und Andererseits den Randbereich des Objektes O tangieren. Analog dazu wird der linke oder erste Eckpunkt Rl der Trajektorie T ermittelt. Durch die beiden Eckpunkte Rr, Rl der Trajektorie T ergibt sich der Winkelbereich WB in dem eine Sequenz von Röntgenaufnahmen für die Tomosynthese TS mit einer maximalen Tiefenauflösung aufgenommen werden können. Ist die oder der Patient bereits bei einer Voruntersuchung gewesen, liegen Eckdaten für Kompressionskraft und der daraus ableitbaren Brustdicke sowie Brustfläche vor. Aufgrund der Position des Objektes O auf der Detektorfläche DF, des 3D-Modells des Objektes O, der Ausmaße der Detektorfläche DF sowie des Abstandes der Röntgenröhre R zur Detektoroberfläche DF kann ausgehend von der Röntgenröhre R eine die Röntgenröhre und den Randbereich des Detektors D verbindende Gerade G, G' gelegt werden, wobei diese Gerade eine Tangente mit dem Objekt bildet.

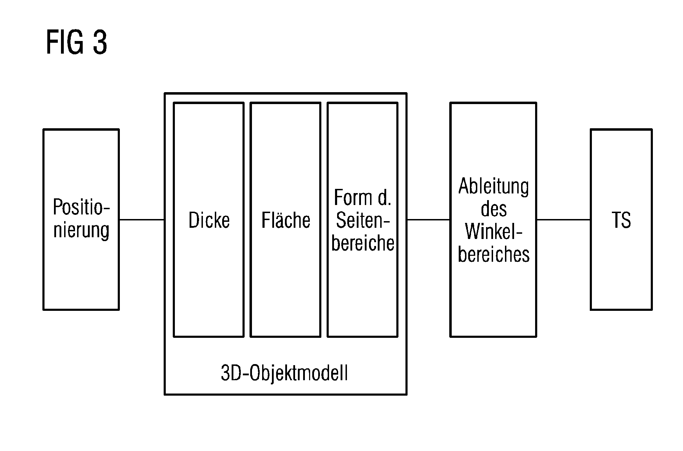

In Figur 3 ist ein Blockdiagramm zur Ermittlung des optimalen Tomosynthesewinkels WB wiedergegeben. In dem ersten Block sind Verfahrensschritte für die Ermittlung der Orientierung und Fixierung des Objektes zusammengefasst. In dem zweiten Block sind Verarbeitungseinheiten sowie Verfahrensschritte für die Erstellung eines individuellen 3D-Brustmodells wiedergegeben. Nachdem das Objekt O durch Komprimierung auf der Detektoroberfläche DF fixiert wurde, wird der Abstand von der Kompressionsplatte KP zur Detektoroberfläche DF mittels Sensoreinheiten erfasst und zwischengespeichert. Nachfolgend wird die Auflagefläche des Objektes beispielsweise über Drucksensoren ermittelt und ebenfalls zwischengespeichert. Ebenfalls könnte ein 3D-Modell aus Voraufnahme und Brustdicke bestimmt werden. In einem nachfolgenden Arbeitsschritt wird die Krümmung der Oberfläche des Objektes O zwischen Kompressionsplatte KP und Detektoroberfläche DF ermittelt. Die Krümmung der Oberfläche kann mit einer Entfernungsbildkamera auf Basis von Infrarotlaufzeitmessungen erfasst werden. Die Entfernungsdaten können beispielsweise mit einem Photonen-Misch-Detektor PMD ermittelt werden. Der PMD Sensor ist ein flächenhaft, räumlich auflösender optischer Abstandssensor, welcher den Abstand zu dem Objekt mit Hilfe von z.B. Infrarotlicht misst. Mit Hilfe des Prinzips des Echolaufzeitverfahrens wird beispielsweise die Oberflächenkrümmung des Objektes O erfasst.

In einem diesen drei Verarbeitungsprozeduren nachgeordneten Modul werden die ermittelten Messdaten zusammengefasst und ein 3D-Modell des Objektes O erstellt.

In dem nachfolgenden Verarbeitungsblock wird eine Tangente G, G' an die konkav verlaufende Oberfläche des Objektes gelegt, wobei der Ausgangspunkt der Tangente G, G' durch den noch aktiven Rand des Detektors D gebildet wird. Der eingeschlossene Winkel A, A' zwischen der Tangenten G, G' und einer auf der Detektoroberfläche DF virtuell errichteten Senkrechten S ergibt die maximale Auslenkung des Röntgenkopfes R. Nach der Ermittlung der links- und rechtsseitigen Eckpunkte Rl, Rr für die Auslenkung des Röntgenkopfes R können bei einer vorgebaren Anzahl von Röntgenaufnahmen die Abstände für die Röntgenaufnahme festgelegt werden in denen bei einem Abfahren der Trajektorie T der Röntgenkopf angehalten wird. Zusätzlich kann je nach Anzahl der Röntgenaufnahmen die Strahlenbelastung für den Patienten verändert werden. Im Block TS werden die Rechenprozeduren der Tomosynthese-Rekonstruktionalgorithmen angestoßen und die Schichtbilder für den Volumensatz erstellt. Die Schichtbilder können dann interaktiv aus Zwischenspeichern der Recheneinheit RE abgerufen und am Bildschirm B ausgegeben werden.

### Bezugszeichenliste

- Kp: Kompressionsplatte
- D: Detektor/Aufnahmeeinheit
- DF: Detektorfläche
- RA: Röntgenabbildung
- PV: Projektionsfläche
- DPF: projizierte Fläche auf der Detektoroberfläche
- OF: reelle Fläche
- R: Röntgenquelle
- RK: Röntgenkegel
- WB: Winkelbereich
- Rr: Röntgenquelle rechtsseitig / zweiter Eckpunkt
- Rl: Röntgenquelle linksseitig / erster Eckpunkt
- O: Objekt
- MO: Modell für Objekt
- OD: Objektdicke
- DM: mittlere Querschnittsfläche
- K: Komprimierung der Brust erstes Modul
- WB: Winkelbereich / drittes Modul
- RE: Rekonstruktion
- G,G': Tangente/Gerade
- S: Senkrechte auf Detektorfläche
- A,A': eingeschlossener Winkel
- E: Eingabeeinheit
- B: Bildschirm
- RE: Recheneinheit
- M1: erstes Modul
- M2: zweites Modul
- M3: drittes Modul
- M4: viertes Modul
- M5: Eckpunktermittlungsmodul
- T: Trajektorie
- TS: Tomosynthese

## Patentansprüche

1. Vorrichtung zur Erstellung eines Volumensatzes aus Röntgenbildern mit einem entlang einer Trajektorie (T) verfahrbaren Röntgenkopf (R) und einer Aufnahmeeinheit (D) zur Aufzeichnung der von einem Objekt (O) gemachten Röntgenaufnahmen,
**gekennzeichnet dadurch,**
**dass** ein Eckpunktermittlungsmodul (M5) zur Ermittlung von Eckpunkten (R1, Rr) der Trajektorie (T) vorgesehen ist, wobei ein Eckpunkt der Trajektorie (T) des Röntgenkopfes (R) dann vorliegt, wenn ein auf dem Röntgenkegel liegender Röntgenstrahl eine zwischen Röntgenkopf (R) und Aufnahmeeinheit (D) liegende Tangente (G, G') an dem Objekt (O) bildet und von der Aufnahmeeinheit (D) erfasst wird.

2. Vorrichtung nach Patentanspruch 1,
**dadurch gekennzeichnet,**
**dass** das Eckpunktermittlungsmodul (M5) zur Ermittlung der Eckpunkte (Rr, Rl) der Trajektorie (T) durch Auslenkung des Röntgenkopfes ( R) derart ausgebildet ist, dass ein Röntgenstahl eines von einem Röntgenkopf (R) ausgehenden Röntgenkegels die Oberfläche des Objektes ( O) tangiert und von der als digitalen Detektor ausgebildeten Aufnahmeeinheit (D) noch erfasst wird.

3. Vorrichtung nach Patentanspruch 1 oder 2,
**dadurch gekennzeichnet,**
**dass** das Eckermittlungsmodul (M5) derart ausgebildet ist, dass zur Ermittlung eines Eckpunktes (R1, Rr) der Trajektorie (T) der Röntgenstrahl betrachtet, der bei einer Projektion eines verlängerten Trajektorieverlaufes auf dem Röntgenkegel überlagert wird.

4. Vorrichtung nach einem der vorhergehenden Patentansprüche,
**dadurch gekennzeichnet,**
**dass** ein drittes Modul (M3) Mittel zur Abtastung und Auswertung der Form des Objektes (O) aufweist.

5. Vorrichtung nach Patentanspruch 4,
**dadurch gekennzeichnet,**
**dass** das Mittel zur Abtastung ein PMD-Sensor ist und mit diesem das Objekt (O) abgetastet und ein 3D-Modell erstellt wird.

6. Vorrichtung nach Patentanspruch 4,
**dadurch gekennzeichnet,**
**dass** das dritte Modul (M3) die Form des Objektes auf der Grundlage von in einer Datenbank hinterlegten 3D-Modellen unter Berücksichtigung von ermittelten Werten ermittelt wird.

7. Vorrichtung nach Patentanspruch 6,
**dadurch gekennzeichnet,**
**dass** das 3D-Modell des Objektes (O) aus einem deformierbaren 3D-Modell aufgrund der ermittelten Werte aus Objektfläche, Kompressionsdicke und Kompressionskraft erstellt wird.

8. Verfahren zur Erstellung eines Volumensatzes mit einem entlang einer Trajektorie (T) verfahrbaren Röntgenkopf (R) und einem Detektor (D) aus dem die von einem Objekt (O) gemachten Röntgenaufnahmen ausgelesen werden,
**gekennzeichnet dadurch,**
**dass** Eckpunkten (R1, Rr) der Trajektorie (T) ermittelt werden, wobei ein Eckpunkt der Trajektorie (T) des Röntgenkopfes (R) dann vorliegt, wenn ein auf dem Röntgenkegel liegender Röntgenstrahl eine zwischen Röntgenkopf (R) und Aufnahmeeinheit (D) liegende Tangente (G, G') an dem Objekt (O) bildet und von der Aufnahmeeinheit (D) erfasst wird.

9. Verfahren nach Patentanspruch 8,
**dadurch gekennzeichnet,**
**dass** die Eckpunkte (Rr, Rl) der Trajektorie (T) durch Auslenkung des Röntgenkopfes ( R) derart ermittelt werden, dass ein Röntgenstahl eines von einem Röntgenkopf (R ) ausgehenden Röntgenkegels die Oberfläche des Objektes ( O) tangiert und von der als digitalen Detektor ausgebildeten Aufnahmeeinheit (D) noch erfasst wird.

10. Verfahren nach Patentanspruch 8 oder 9,
**dadurch gekennzeichnet,**
**dass** zur Ermittlung eines Eckpunktes (R1, Rr) der Trajektorie (T) der Röntgenstrahl auf dem Röntgenkegel betrachtet wird, der in der Fortführung der Trajektorie (T) auf dem Röntgenkegel liegt.

11. Verfahren nach einem der vorhergehenden Patentansprüche,
**dadurch gekennzeichnet,**
**dass** die Form des Objektes (O) abgetastet wird.

12. Verfahren nach einem der vorhergehenden Patentansprüche,
**dadurch gekennzeichnet,**
**dass** die Form des Objektes (O) aus einem 3D-Modell-Datenbank bestimmt wird, die eine Objektfläche, Kompressionsdicke und Kompressionskraft berücksichtigt.

13. Verfahren nach einem der vorhergehenden Patentansprüche,
**dadurch gekennzeichnet,**
**dass** die Form des Objektes (O) aus einem deformierbaren 3D-Modell bestimmt wird, die die Objektfläche, Kompressionsdicke und Kompressionskraft berücksichtigt.
